**Europäisches Patentamt**

**European Patent Office**

**Office européen des brevets**

(19)

(11) Veröffentlichungsnummer: **0 022 523**
**B1**

# EUROPÄISCHE PATENTSCHRIFT

(12)

(45) Veröffentlichungstag der Patentschrift:
02.06.82    .

(21) Anmeldenummer: **80103792.0**

(22) Anmeldetag: **04.07.80**

(51) Int. Cl.³: **C 07 D 213/53**

(54) **Verfahren zur Herstellung von Pyridin-4-aldehyd-phenylhydrazonen.**

(30) Priorität: **17.07.79 DE 2928746**

(43) Veröffentlichungstag der Anmeldung:
**21.01.81 Patentblatt 81/3**

(45) Bekanntmachung des Hinweises auf die Patenterteilung:
**02.06.82 Patentblatt 82/22**

(84) Benannte Vertragsstaaten:
**CH DE FR GB LI**

(56) Entgegenhaltungen:
**DE-AS-1 215 155**
**DE-AS-1 215 156**
**US-PS-2 945 862**
**JOURNAL OF ORGANIC CHEMISTRY,**
**Band 36, Nr. 23, 1971,**
**Columbus, Ohio, US,**
**W. W. ZAJAC et al.: »Reaction of Nitriles with Hydrazine Hydrate and Raney Nickel«, Seiten 3539—41**

(73) Patentinhaber: **BAYER AG, Zentralbereich Patente, Marken und Lizenzen, D-5090 Leverkusen 1, Bayerwerk (DE)**

(72) Erfinder: **Giesecke, Heinz, Dr., Am Hang 19, D-5090 Leverkusen 3 (DE)**
Erfinder: **Brack, Alfred, Dr., Auf dem Krahwinkel 7, D-5068 Odenthal (DE)**

## Verfahren zur Herstellung von Pyridin-4-aldehyd-phenylhydrazonen

Gegenstand der Erfindung ist ein Verfahren zur Herstellung von Pyridin-4-aldehyd-phenylhydrazonen durch drucklose oder nahezu drucklose katalytische Hydrierung von 4-Cyan-pyridin in Gegenwart von Phenylhydrazin oder dessen Kernsubstitutionsprodukten in wäßrigem Milieu in Abwesenheit von organischen Lösungsmitteln bei einem pH-Wert zwischen 9—12, vorzugsweise 9,5—11, und einem Temperaturbereich von 0 bis 60°C. Die alkalische Reaktion wird durch Zugabe von vorzugsweise Ammoniak oder anderen alkalischen Mitteln erreicht. Das erfindungsgemäße Verfahren zeichnet sich durch besondere Einfachheit, Sicherheit und Wirtschaftlichkeit aus. So wird die Verwendung brennbarer oder toxischer Lösungsmittel vermieden, und es ist keine aufwendige Druckapparatur erforderlich.

Pyridin-4-aldehyd-phenylhydrazon ist bisher aus Pyridin-4-aldehyd hergestellt worden, der u. a. auch durch Säurehydrolyse des durch Hydrieren von Cyanpyridin erhaltenen Aldimins zugänglich ist. Diese Säurehydrolyse wird üblicherweise mit starken Säuren, wie Schwefelsäure. durchgeführt. Unter Druck kann auch mit Kohlensäure hydrolysiert werden. Das vorliegende Verfahren erlaubt den Verzicht auf diesen Reaktionsschritt und macht Pyridin-4-aldehyd-phenylhydrazone nicht nur ohne Zwischenisolierung, sondern überhaupt ohne intermediäre Erzeugung des empfindlichen Pyridin-4-aldehyds zugänglich.

Das neue Verfahren stellt also eine wesentliche Vereinfachung der vorbekannten Methoden zur Herstellung von Pyridin-4-aldehyd-phenylhydrazonen dar und überwindet zugleich das bisher offensichtlich bestehende Vorurteil, daß zur Vermeidung von Sekundärreaktionen die partielle Hydrierung von Nitrilen bis zur Oxidationsstufe des Aldehyds nur in Kombination mit der momentanen Hydrolyse des sehr reaktiven Aldimins brauchbar ist.

Als Kernsubstituenten des Phenylhydrazins kommen die in der Chemie der kationischen Farbstoffe üblichen nichtionischen Reste, wie niedere Alkyl- und Alkoxygruppen sowie Halogenatome, vorzugsweise Methyl, Ethyl, Methoxy, Ethoxy, Fluor und Chlor in Frage.

Geeignete Phenylhydrazine sind demzufolge neben Phenylhydrazin selbst z. B. 2-, 3- und 4-Methyl-phenylhydrazin, 2-, 3- und 4-Methoxy- und Ethoxy-phenylhydrazin, 2-, 3- und 4-Fluor- und 2-, 3- und 4-Chlor-phenylhydrazin, 2,4- und 2,4-Dichlor-phenylhydrazin und 2,4-Dimethoxy- und Diethoxy-phenylhydrazin.

An alkalischen Mitteln sind neben Ammoniak verwendbar z. B. Natronlauge, Kalilauge, Kalkmilch, Sodalösung und Puffermischungen im angegebenen pH-Bereich, wie Phosphat- und Borat-Puffer.

Pyridin-4-aldehyd-phenylhydrazone sind wertvolle Zwischenprodukte zur Herstellung von kationischen Farbstoffen, wie sie z. B. in der DE-PS 1 133 054 beschrieben sind.

Geeignete Katalysatoren sind beispielsweise feinverteiltes Palladium oder Platin, gegebenenfalls auf einem geeigneten Trägermaterial, sowie nickelhaltige Skelettkatalysatoren, von denen Raney-Nickel bevorzugt ist.

### Beispiel 1

Man trägt bei Raumtemperatur in 200 ml 25%ige wäßrige Ammoniaklösung 27,8 g Phenylhydrazinhydrochlorid ein und fügt 20 g 4-Cyanpyridin hinzu. Nach Zugabe von 100 ml 20%iger Salzsäure läßt man unter Rühren auf 25°C abkühlen, versetzt mit 4 g wasserfeuchtem Raney-Nickel und hydriert bei Raumtemperatur und normalem Druck auf einer Schüttelapparatur, bis das 1,5fache der theoretisch benötigten Wasserstoffmenge aufgenommen ist.

Nach Absaugen des Pyridin-4-aldehyd-phenylhydrazon/Raney-Nickel-Gemisches wäscht man mit 100 ml Wasser und saugt erneut scharf ab.

Ausbeute: 30,5 g des feuchten Gemisches aus Pyridin-4-aldehyd-phenylhydrazon und Raney-Nickel.

Durch Ausrühren mit Aceton, Filtrieren vom Nickel und Eindampfen des Filtrats zur Trockne erhält man daraus 56—58% der Theorie reines Pyridin-4-aldehyd-phenylhydrazon.

### Beispiel 2

Man gibt zu 127 g 25%iger wäßriger Ammoniaklösung 43,2 g Ammoniumchlorid, tropft anschließend bei 20°C 20,8 g Phenylhydrazin zu und gibt 20,0 g 4-Cyan-pyridin zu der resultierenden Lösung. Nach Zugabe von 4 g wasserfeuchtem Raney-Nickel hydriert man auf einer Schüttelapparatur bei Raumtemperatur und Normaldruck, bis ca. das 1,3fache der theoretisch benötigten Wasserstoffmenge aufgenommen ist.

Aufarbeitung nach Art des Beispiels 1 ergibt Pyridin-4-aldehyd-phenylhydrazon in vergleichbarer Ausbeute und Reinheit.

### Beispiel 3

Man gibt zu 127 g 25%iger wäßriger Ammoniaklösung 43,2 g Ammoniumchlorid und fügt bei Raumtemperatur 27,4 g 4-Chlor-phenylhydrazin zu. Nach weiterer Zugabe von 20,0 g 4-Cyan-pyridin und 4 g wasserfeuchtem Raney-Nickel hydriert man auf einer Schüttelapparatur bei Raumtemperatur und Normaldruck, bis etwa das 1,5fache der theoretisch benötigten Wasserstoffmenge aufgenommen worden ist. Nach Absaugen des Katalysator/Hydrazon-Gemisches wäscht man mit 100 ml Wasser, saugt ab, gibt zum feuchten Filterkuchen 200 ml Toluol und destilliert ca. 80 ml ab. Die zurückbleibende Toluollösung scheidet beim Erkalten Pyridin-4-aldehyd-(4-chlorphenyl)-hydrazon in guter Ausbeute und Reinheit aus.

Bei Verwendung von 4-Methyl-phenylhydrazin, 4-Ethyl-phenylhydrazin, 4-Methoxy- oder 4-Ethoxy-phenylhydrazin anstelle von 4-Chlorphenylhydrazin erhält man bei im übrigen unveränderter Arbeitsweise die entsprechenden Hydrazone in ähnlicher Ausbeute und Reinheit.

**Patentansprüche**

1. Verfahren zur Herstellung von Pyridin-4-aldehyd-phenylhydrazonen, die im Phenylkern durch nichtionische Substituenten substituiert sein können, dadurch gekennzeichnet, daß man 4-Cyan-pyridin in wäßrigem Milieu unter normalem Druck bei einem pH-Wert zwischen 9 und 12 in Anwesenheit von Phenylhydrazin oder dessen im Phenylkern durch nichtionische Reste substituierten Derivaten bei Temperaturen von 0—60°C katalytisch hydriert.

2. Verfahren zur Herstellung von Pyridin-4-aldehyd-phenylhydrazonen, die im Phenylkern durch eine oder zwei Methyl-, Ethyl-, Methoxy- oder Ethoxygruppen, Fluor- oder Chloratome substituiert sein können, dadurch gekennzeichnet, daß man 4-Cyan-pyridin in Gegenwart von Phenylhydrazin oder dessen im Phenylkern durch eine oder zwei Methyl-, Ethyl-, Methoxy- oder Ethoxygruppen, Fluor- oder Chloratome substituierten Derivaten gemäß Anspruch 1 katalytisch hydriert.

3. Verfahren zur Herstellung von Pyridin-4-aldehyd-phenylhydrazonen, die im Phenylkern durch eine 4ständige Methyl-, Methoxy- oder Ethoxygruppe substituiert sein können, dadurch gekennzeichnet, daß man 4-Cyanpyridin in Gegenwart von Phenylhydrazin, 4-Methyl-, 4-Methoxy- oder 4-Ethoxyphenylhydrazin gemäß Anspruch 1 katalytisch hydriert.

4. Verfahren nach den Ansprüchen 1 bis 3, dadurch gekennzeichnet, daß man als Katalysator Raney-Nickel verwendet.

**Claims**

1. Process for the preparation of pyridine-4-aldehyde phenylhydrazones which can be substituted in the phenyl nucleus by non-ionic substituents, characterised in that 4-cyano-pyridine is catalytically hydrogenated in an aqueous medium under normal pressure at a pH value between 9 and 12 in the presence of phenylhydrazine or derivatives thereof substituted in the phenyl nucleus by non-ionic radicals, at temperatures of 0—60°C.

2. Process for the preparation of pyridine-4-aldehyde phenylhydrazones which can be substituted in the phenyl nucleus by one or two methyl, ethyl, methoxy or ethoxy groups or fluorine or chlorine atoms, caracterised in that 4-cyano-pyridine is catalytically hydrogenated, according to Claim 1, in the presence of phenylhydrazine or derivatives thereof substituted in the phenyl nucleus by one or two methyl, ethyl, methoxy or ethoxy groups or fluorine or chlorine atoms.

3. Process for the preparation of pyridine-4-aldehyde phenylhydrazones which can be substituted in the phenyl nucleus by a methyl, methoxy or ethoxy group in the 4-position, characterised in that 4-cyano-pyridine is catalytically hydrogenated, according to Claim 1, in the presence of phenylhydrazine or 4-methyl-, 4-methoxy- or 4-ethoxy-phenylhydrazine.

4. Process according to Claims 1 to 3, characterised in that Raney nickel is used as the catalyst.

**Revendications**

1. Procédé pour la préparation de phénylhydrazones de pyridine-4 aldéhyde qui peuvent être substituées au noyau phényle par des substituants non ioniques, caractérisé en ce que l'on hydrogène catalytiquement la 4-cyanopyridine en milieu aqueux sous pression normale, à un pH entre 9 et 12, en présence de phénylhydrazine ou de ses dérivés substitués au noyau phényle par des restes non ioniques à des températures de 0—60°C.

2. Procédé pour la préparation de phénylhydrazones de pyridine-4 aldéhyde qui peuvent être substituées au noyau phényle par un ou deux groupes méthyle, éthyle, méthoxy ou éthoxy ou atomes de fluor ou de chlore, caractérisé en ce que l'on hydrogène la 4-cyanopyridine selon la revendication 1, en présence de phénylhydrazine ou de ses dérivés substitués au noyau phényle par un ou deux groupes méthyle, éthyle, méthoxy ou éthoxy ou atome de fluor ou de chlore.

3. Procédé pour la préparation de phénylhydrazones de pyridine-4 aldéhyde qui peuvent être substituées au noyau phényle par un groupe phényle, méthoxy ou éthoxy en position 4, caractérisé en ce que l'on hydrogène catalytiquement selon la revendication 1 la 4-cyanopyridine en présence de phénylhydrazine, de 4-méthyl-, 4-méthoxy- ou 4-éthoxyphénylhydrazine.

4. Procédé selon les revendications 1 à 3, caractérisé en ce que l'on utilise comme catalyseur le nickel de Raney.